# EUROPEAN PATENT APPLICATION

(11) **EP 1 048 300 A1**
(43) Date of publication of application: **02.11.2000**
(21) Application number: 99900297.5
(22) Date of filing: 13.01.1999
(51) Int. Cl.: A61K 47/26

(54) **DISINTEGRATING AGENT**

(30) Priority: 14.01.1998 JP 561098
(71) Applicant: DAIICHI PHARMACEUTICAL CO., LTD., Chuo-ku, Tokyo 103-8234 (JP)
(72) Inventor: MURAKAMI, Toshio, Daiichi Pharm. Co., Ltd., Tokyo 134-8630 (JP); II, Noritaka, Daiichi Pharmaceutical Co., Ltd., Tokyo 134-8630 (JP); SAKURAI, Hiroyuki, Daiichi Pharmaceutical Co., Ltd, Tokyo 134-8630 (JP)
(74) Representative: Hartz, Nikolai F., Dr.
(86) International application number: JP9900083
(87) International publication number: WO9936097

(57) **Abstract**

The present invention relates to a disintegrant comprising a substance which is solid at room temperature and has a water solubility of 30 wt.% or more, a saturated aqueous solution of the substance having a viscosity of 50 mPa·s.

## Description

### Technical Field

The present invention relates to a disintegrant which is incorporated into solid compositions used in the fields of drug products, health food products, etc.

### Background Art

Orally administered drugs or health foods exhibit their effects after they reach the digestive organs, at which point pharmaceutically active ingredients or nutritional ingredients contained therein are absorbed through the organs and then distributed within the body. Examples of product forms for oral administration include tablets, capsules, granules, fine granules, pills, and powders. Many modifications have been made to these product forms in order to enhance absorbability of pharmaceutically active ingredients or nutritional ingredients in the body and to improve sensation upon taking of the products.

When a pharmaceutical composition has poor disintegrability, elution of pharmaceutically active ingredients therefrom within the digestive organs is unsatisfactory, reducing the absorbability of the ingredients. In order to enhance disintegrability of such a composition, a water-swellable disintegrant is incorporated into the composition. Examples of widely-used water-swellable disintegrants include low substituted degree hydroxypropylcellulose, crosscarmellose sodium, carmellose, and carmellose calcium.

Such a water-swellable disintegrant is water-insoluble, but the volume thereof increases when the agent absorbs moisture or comes into contact with water. Therefore, in order to incorporate the agent into a solid composition such as a tablet, the composition must be designed in consideration of reduction in hardness of a tablet or increase in volume of the composition due to moisture absorption. Particularly, in the case of film-coated tablets and sugar-coated tablets, if a water-swellable disintegrant is incorporated into the composition before coating, the tablets may absorb moisture with passage of time, resulting in cracking or breakage of a film-coated layer or sugar-coated layer. Incidentally, cellulose is a fibrous substance having a relatively large particle size. Therefore, a composition containing cellulose is disadvantageous, in that it is apt to provide a gritty sensation in the oral cavity upon administration, causing an unfavorable sensation upon oral administration. Particularly, cellulose exhibits such adverse effects on powders, granules, or shaped products which rapidly disintegrate or dissolve in the oral cavity.

In order to improve disintegrability of a solid composition, the aforementioned water-swellable disintegrant is generally incorporated therein. Another known method to improve disintegrability is addition of a surfactant, which enhances affinity of the composition to water (i.e., improvement in wetting of the composition). However, a surfactant may cause problems in terms of safety, and thus is not a preferable additive.

In view of the foregoing, an object of the present invention is to provide a novel disintegrant in which the aforementioned drawbacks are avoided and which can replace a water-swellable disintegrant that deteriorates the stability of a solid composition containing the agent with passage of time due to moisture absorption.

### Disclosure of the Invention

The present inventors have performed extensive studies, and have found that a substance which is solid at room temperature and has a water solubility of 30 wt.% or more at 37°C, a saturated aqueous solution of the substance having a viscosity of 50 mPa·s or less at 37°C, can be employed as a new disintegrant. The present invention has been accomplished on the basis of this finding.

Accordingly, the present invention provides a disintegrant comprising a substance which is solid at room temperature and has a water solubility of 30 wt.% or more at 37°C, a saturated aqueous solution of the substance having a viscosity of 50 mPa·s or less at 37°C, and a solid composition comprising the disintegrant.

### Brief Description of the Drawings

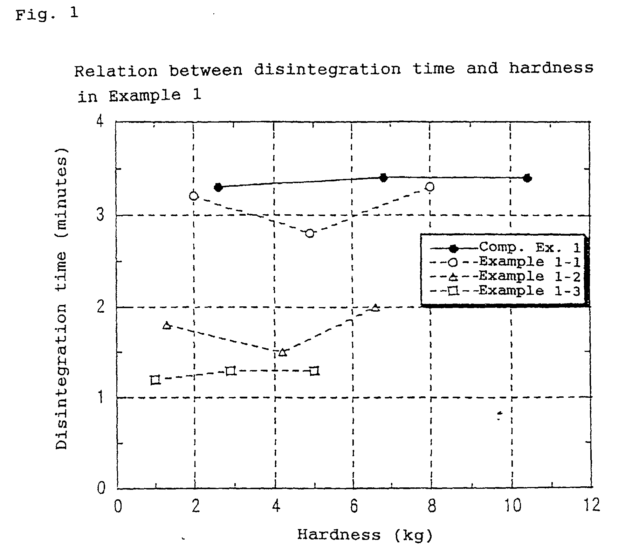

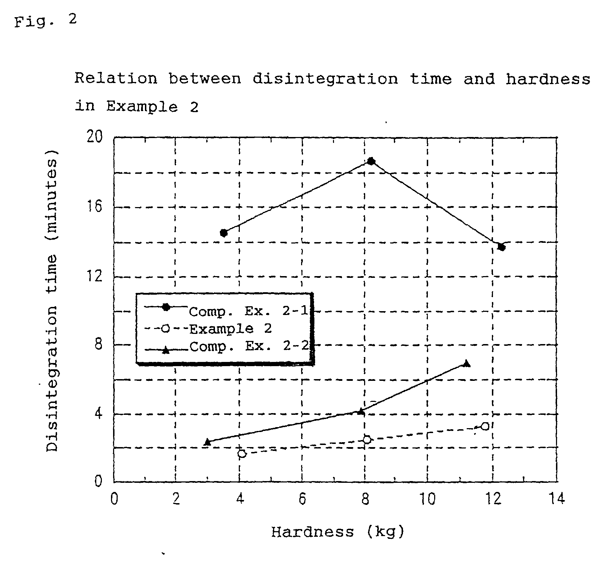

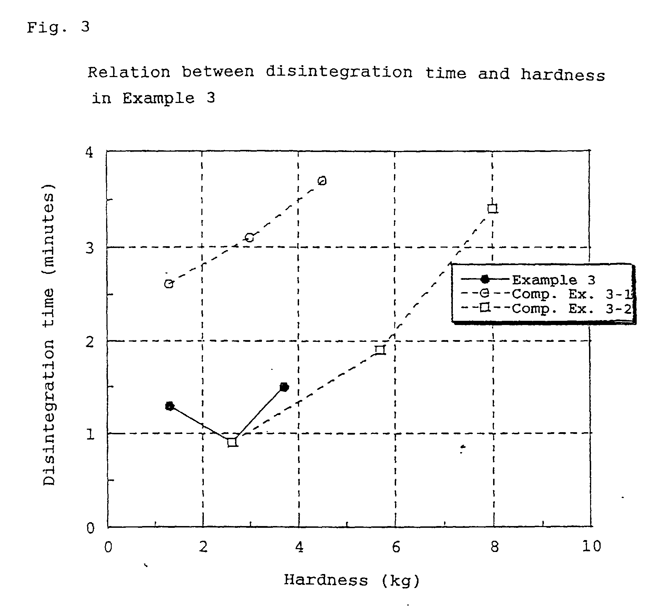

Fig. 1 is a graph showing the relation between disintegration time (i.e., time required for disintegration) and hardness in Examples 1-1 through 1-3 and Comparative Example 1. Fig. 2 is a graph showing the relation between disintegration time and hardness in Example 2 and Comparative Examples 2-1 and 2-2. Fig. 3 is a graph showing the relation between disintegration time and hardness in Example 3 and Comparative Examples 3-1 and 3-2.

### Best Mode for Carrying Out the Invention

The term "the disintegrant of the present invention" refers to a disintegrant comprising a substance which is solid at room temperature and has a water solubility of 30 wt.% or more at 37°C, a saturated aqueous solution of the substance having a viscosity of 50 mPa·s or less at 37°C. As used herein, the term "room temperature" refers to a temperature of 1-30°C. The disintegrant of the present invention is preferably solid at 30°C.

When the disintegrant of the present invention is incorporated into a solid composition, permeability of water into the composition is enhanced, since the disintegrant of the present invention has high water solubility and high rate of dissolution into water, and a saturated aqueous solution of the agent has low viscosity. The solid composition is considered to disintegrate and dissolve with dissolution of the disintegrant. In addition, the solid composition is stable with passage of time, because the volume of the composition does not increase when the composition absorbs moisture or is brought into contact with water.

Examples of the disintegrant of the present invention include erythritol, trehalose, xylitol, maltose, potassium acetate, sodium acetate, sodium citrate, and dibasic potassium phosphate. Of these, erythritol, trehalose, xylitol, and maltose are preferable. These disintegrants may be employed singly or in combination of two or more species.

Erythritol is a glucose fermentation sweetener, a tetra-valent sugar alcohol, and a white crystalline powder having a melting point of 119°C, and is easily dissolved in water. Erythritol has a heat of dissolution of -42.9 cal/g, provides a cool sensation, and is not hygroscopic. Erythritol is a sweetener having a sweetness of 70-80% that of sucrose. Trehalose (α,α-trehalose) is a white crystalline powder having a melting point of 97°C, is easily dissolved in water, is not hygroscopic (dihydrate crystal), and is a sweetener having a sweetness of approximately 45% that of sucrose. Xylitol is a penta-valent sugar alcohol and a white crystalline powder having a melting point of 93-95°C. Xylitol is very easily dissolved in water, has a heat of dissolution of -35 cal/g, provides a cool sensation, is slightly hygroscopic, and is a sweetener having a sweetness which is equal to that of sucrose. Maltose is a disaccharide consisting of two glucose molecules and a white crystalline powder. The melting points of maltose anhydride and maltose hydrate are 155°C or higher and 120-130°C, respectively. Maltose is easily dissolved in water and is a sweetener having a sweetness of approximately 33% that of sucrose.

The disintegrant of the present invention is appropriately incorporated into a solid composition in an amount of 5-99 wt.% on the basis of the entirety of the composition, preferably 10-99 wt.%, more preferably 20-99 wt.%. When the amount is less than 5 wt.%, the effect of the agent for ameliorating disintegration or dissolution of the composition is insufficient, resulting in poor disintegrability and solubility of the composition.

Erythritol, trehalose, xylitol, and maltose can be employed as an excipient, and thus even when they are incorporated into a solid composition in large amounts, no problem arises in the composition. The greater the amount of these agents contained in a solid composition, the more enhanced the effect of the agents for ameliorating disintegration or dissolution of the composition. However, when erythritol is incorporated into a tablet, the amount of erythritol is appropriately 80 wt.% or less, because when erythritol is incorporated into a tablet in large amounts, shapability of the tablet may deteriorate, which causes the tablet to have low hardness.

The disintegrant of the present invention exhibits effects for ameliorating disintegrability or solubility of a solid composition. Particularly, the agent is suitably employed for ameliorating disintegrability of a crude film-coated tablet or sugar-coated tablet. For example, erythritol has no hygroscopicity or swellability, and thus even when erythritol is incorporated into a crude film-coated or sugar-coated tablet in large amounts, the tablet does not undergo cracking or breakage due to moisture absorption with passage of time. When erythritol is incorporated into a solid composition together with a conventionally-employed water-swellable disintegrant such as low subtituted hydroxypropylcellulose, the amount of such a conventional water-swellable disintegrant can be reduced. Therefore, the size of the composition can be reduced.

The disintegrant of the present invention is effectively incorporated into powders, granules, chewable tablets, or shaped products which rapidly disintegrate or dissolve in the oral cavity. Namely, the disintegrant of the present invention, which differs from conventionally-employed cellulose such as low substituted hydroxypropylcellulose, is not a fibrous substance, and thus a solid composition containing the agent does not provide a gritty sensation in the oral cavity. In addition, the composition dissolves rapidly in the oral cavity, and the composition can provide a favorable sensation on oral administration.

In the present invention, the product shape of a solid composition is not particularly limited. Examples of the product shape include tablet, troche, capsule, granule, powder, and pill. Examples of tablets include chewable tablets, effervescent tablets, and shaped products which dissolve and disintegrate in the oral cavity and which can be administered orally without aid of water. Examples of granules and powders include dry syrups which are dissolved upon use and granular products which dissolve and disintegrate in the oral cavity and can be administered orally without aid of water.

The disintegrant of the present invention may be incorporated into drug products and health food products. No particular limitation is imposed on the species of pharmaceutically active ingredients or nutritional ingredients contained in drugs and health foods according to purposes. These ingredients may take any form, such as powder, crystal, oil, or solution.

Examples of ingredients contained in drug products and health food products include vitamin A, vitamin B₁ (e.g., thiamin hydrochloride), vitamin B₂, vitamin B₆, vitamin B₁₂, vitamin C (e.g., ascorbic acid, sodium ascorbate), vitamin D, vitamin E, nicotinamide, calcium pantothenate, pantethine, epsilon aminocapronic acid, tranexamic acid, gamma aminobutyric acid, carpronium chloride, procainamide hydrochloride, alimemazine tartrate, isoniazid, pilsicainide hydrochloride, ticlopidine hydrochloride, cinepazide maleate, sulpyrine, aspirin, acetaminophen, ethenzamide, ibuprofen, ketoprofen, indomethacin, cimetidine, famotidine, caffeine, ofloxacin, levofloxacin, nalidixic acid, carvedilol, sulfadimethoxine, reserpine, lofepramine hydrochloride, malotilate, baclofen, probucol, sulfamonomethoxine, levodopa, timiperone, cetraxate hydrochloride, flopropione, budralazine, oxypertine, and epirizol. Pharmaceutically active ingredients and nutritional ingredients may be incorporated into a solid composition singly or in combination of two or more species.

Generally-employed various composition additives may further be incorporated into a solid composition comprising the disintegrant of the present invention, so long as such additives do not impede the effect of the disintegrant (e.g., shortening of disintegration time, enhancement of stability with passage of time). Examples of composition additives include excipients, disintegrants, binders, lubricants, coloring agents, sweeteners, and sweetening agents. Specific examples of these additives will next be described.

Examples of excipients include water-soluble excipients such as lactose, sucrose, fructose, glucose, mannitol, sorbitol, macrogol, powder hydrogenated maltose starch syrup, and hydrogenated lactose, and water-insoluble excipients such as corn starch, potato starch, wheat starch, rice starch, crystalline cellulose, light anhydrous silicic acid, dried aluminum hydroxide gel, magnesium aluminosilicate, calcium silicate, synthetic aluminum silicate, synthetic hydrotalcite, hydrate silicon dioxide, magnesium oxide, magnesium hydroxide, calcium carbonate, and calcium hydrogenphosphate.

Examples of disintegrants include starches such as partially pregelatinized starch, hydroxypropyl starch, and sodium carboxymethyl starch; celluloses such as crystalline cellulose, powder cellulose, low substituted hydroxypropylcellulose, carmellose, carmellose calcium, croscarmellose sodium, and carboxymethylethylcellulose; polymer compounds such as alginic acid, guar gum, casein formamide, pectin, ion exchange resin, cross-linking polyvinylpyrrolidone; and inorganic substances such as bentonite (colloidal hydrated aluminum silicate) and beegum (a mixture of magnesium silicate and aluminum silicate).

Examples of binders include methylcellulose, hydroxypropylcellulose, hydroxypropylmethylcellulose, polyvinyl alcohol, and polyvinylpyrrolidone.

Examples of lubricants include magnesium stearate, calcium stearate, talc, and sucrose esters of fatty acids.

Examples of coloring agents include food yellow No. 5, food dye red No. 2, food dye blue No. 2, food lake dye, yellow ferric oxide, and titanium oxide.

Examples of sweeteners include Aspartame, Stevia, sormatin, sodium saccharin, and dipotassium glycyrrhetinate.

Examples of sweetening agents include L-menthol, camphor, peppermint, sodium L-glutamate, disodium inosinate, and magnesium chloride.

These composition additives may be appropriately incorporated into a solid composition during a suitable process in the course of production of the solid composition.

A solid composition comprising the disintegrant of the present invention may be produced through a known process for producing a solid composition. Examples of granulation methods which may be employed include a fluidized-bed granulation, an agitating granulation, an agitating fluidized-bed granulation, an extrusion granulation, a spray granulation, and a pulverization granulation.

An example process for producing a solid composition by means of a fluidized-bed granulation method will next be described.

Erythritol and, if desired, excipients such as lactose and corn starch are incorporated into pharmaceutically active ingredients and/or nutritional ingredients, and the resultant mixture is granulated by use of an aqueous solution of a binder such as hydroxypropylcellulose or polyvinyl alcohol through a fluidized-bed granulation-drying machine. If desired, a sweetener such as Aspartame is added to the granules, and mixed by means of a mixing machine to produce granules, powders, or fine granules. Incidentally, a lubricant such as magnesium stearate or talc may be added to the thus-granulated product in a required amount, and after mixing, the resultant mixture may be tableted by means of a tableting machine, producing tablets or chewable tablets.

### Examples

The present invention will next be described in more detail by way of examples, which should not be construed as limiting the invention thereto.

### 〈Test method〉

The following tests were performed in order to describe the present invention in more detail.

### (1) Measurement of water solubility

A saturated aqueous solution of a sample was prepared at 37°C, and the resultant solution was filtered by use of a membrane filter. A predetermined volume of the filtrate was precisely weighed and dried by means of a freeze-drying method, so that the water content was obtained. Water solubility was calculated on the basis of the thus-obtained water content.

### (2) Measurement of viscosity of a saturated aqueous solution

A saturated aqueous solution of a sample was prepared at 37°C, and the resultant solution was filtered by use of a membrane filter. The viscosity of the solution was measured by use of the resultant filtrate at 37°C by means of a B-type viscometer.

### (3) Hardness of a tablet

The hardness of a tablet in a radial direction was measured by use of a tablet hardness tester (Schleuniger tablet hardness tester, product of Freund Industrial Co., Ltd.). Measurement was performed on five sample tablets, and the mean value is shown in Tables below.

### (4) Disintegration test

According to the disintegration test method of tablets in Pharmacopoeia of Japan (13th edition), measurement was performed on six sample tablets by use of a disintegration tester (product of Toyama Sangyo) without use of a disk. The mean value is shown in Tables below.

### (5) Disintegration test in the oral cavity

Three healthy adult men tested tablets for the time required for complete disintegration of the tablets by saliva in the oral cavity (without aid of water).

### (6) Tableting pressure

Tableting pressure was measured during manufacture of sample tablets, and the mean tableting pressure per punching (kg/punching) of each sample tablet is shown in Tables below.

### (7) Increase in weight by moisture absorption

A sample tablet was weighed before and after moisture absorption, and the increase in weight of the tablet by moisture absorption (%) was calculated.

### (8) Percentage of increase in volume

The volume of a sample tablet was measured before and after moisture absorption, and percentage of increase in volume of the tablet (%) was calculated.

### Test Example 1

In Test Example 1, water solubility of a saturated aqueous solution of erythritol, trehalose, xylitol, maltose, potassium acetate, sodium acetate, or sodium citrate and viscosity of each of the saturated aqueous solutions of these compounds were measured at 37°C. In Reference Example 1, water solubility of a saturated aqueous solution of lactose, D-mannitol, D-sorbitol, hydrogenated maltose starch syrup, hydrogenated lactose, glucose, or sucrose and viscosity of each of the saturated aqueous solutions of these compounds were measured in the same manner as in Test Example 1. The results are shown in Table 1.

**Table 1**

| | Sample | Solubility (37°C) W/V% | Viscosity (37°C) mPa·s |
|---|---|---|---|
| Test Example 1 | Erythritol | 45 | 3.5 |
| | Trehalose | 50 | 11 |
| | Xylitol | 74 | 37 |
| | Maltose | 46 | 38 |
| | Potassium acetate | 76 | 30 |
| | Sodium acetate | 38 | 5.2 |
| | Sodium citrate | 36 | 5.8 |
| Reference Example 1 | Lactose | 25 | 1.7 |
| | D-Mannitol | 24 | 1.6 |
| | D-Sorbitol | 88 or more^{*1} | 2090 or more |
| | Hydrogenated maltose starch syrup | 79 | 488 |
| | Hydrogenated lactose | 74 | 218 |
| | Glucose | 83 | 282 |
| | Sucrose | 78 | 1120 |

| | | | |
|---|---|---|---|
| Note) *1: Preparing a saturated aqueous solution was difficult, due to high water solubility. | | | |

### Test Example 2

In test Example 2, erythritol and trehalose (in the form of hydrous crystals, products of Hayashibara Shoji Inc.) and xylitol and maltose (in the form of anhydrous crystals, products of Nihon Shokuhin Kako Co., Ltd.) were stored for seven days at a temperature of 25°C and a relative humidity of 75%. Thereafter, each of these was subjected to measurement of increase in weight by moisture absorption (%). In Reference Example 2, the increases in weight by moisture absorption (%) of cornstarch, low substituted hydroxypropylcellulose, carmellose, carmellose calcium, and carmellose sodium were measured in the same manner as in Test Example 2. These compounds had been dried in a drier at 80°C for one hour, and then employed as samples.

**Table 2**

| | Sample | 25°C, 75% open air, 7 days |
|---|---|---|
| Test Example 2 | Erythritol | 0.03% |
| | Trehalose | 0.99% |
| | Xylitol | 0.05% |
| | Maltose | 0.06% |
| Reference Example 2 | Corn starch | 8.53% |
| | Low substituted hydroxypropylcellulose | 14.09% |
| | Carmellose | 11.55% |
| | Carmellose calcium | 17.49% |
| | Carmellose Sodium | 21.07% |

As is apparent from Table 2, erythritol, trehalose, xylitol, and maltose absorb little moisture, whereas water-swellable disintegrants in Reference Example 2; i.e., low substituted hydroxypropylcellulose, carmellose, carmellose calcium, and carmellose sodium, absorb moisture, and the increase in weight by moisture absorption ranges from 10 to 20%.

### Example 1

Lactose and corn starch were added into a fluidized-bed granulation-drying machine on the basis of the formulations of Examples 1-1 through 1-3 shown in Table 2, and mixed for three minutes. The resultant mixture was granulated by use of a 5 w/v% aqueous solution (100 ml) of hydroxypropylcellulose (HPC_{L}, product of Nippon Soda Co., Ltd.) under the following conditions: spray pressure 1.5 kg/cm², spray solution rate 15 ml/minute. After being dried, the resultant granules were sieved by use of a 16-mesh sieve (1000 µm). Erythritol [product of Nikken Chemicals Co., Ltd., sieved through 42 mesh (350 µm)] and magnesium stearate were added to the thus-sieved granules on the basis of the formulations of Examples 1-1 through 1-3 shown in Table 3, and mixed. Subsequently, the mixture was prepared into tablets by use of a punch having a flat impact face (diameter: 10 mm) in a single tableting machine at three different tableting pressures (from low to high pressure). The weight of a tablet was 400 mg. In Comparative Example 1, on the basis of the formulation shown in Table 3, tablets were produced in the same manner as in Example 1. The thus-produced tablets were subjected to the disintegration test. The results are shown in Table 4 and Fig. 1.

**Table 3**

| Formulation | Example | | | Comparative Example |
|---|---|---|---|---|
| | 1-1 | 1-2 | 1-3 | 1 |
| Lactose | 247 | 219 | 191 | 275 |
| Corn starch | 106 | 94 | 82 | 118 |
| Erythritol | 40 | 80 | 120 | - |
| Hydroxypropylcellulose | 5 | 5 | 5 | 5 |
| Magnesium stearate | 2 | 2 | 2 | 2 |
| Total | 400 | 400 | 400 | 400 |
| Note) In the formulation, unit is gram (g). | | | | |

**Table 4**

| | Tableting pressure | Weight (g) | Hardness (kg) | Disintegration time (minute) |
|---|---|---|---|---|
| Example 1-1 | 530 | 402 | 2.0 | 3.2 |
| | 1075 | 400 | 4.9 | 2.8 |
| | 1450 | 400 | 8.0 | 3.3 |
| Example 1-2 | 520 | 400 | 1.3 | 1.8 |
| | 1100 | 403 | 4.2 | 1.5 |
| | 1515 | 404 | 6.6 | 2.0 |
| Example 1-3 | 505 | 402 | 1.0 | 1.2 |
| | 1000 | 401 | 2.9 | 1.3 |
| | 1600 | 399 | 5.0 | 1.3 |
| Comparative Example 1 | 525 | 402 | 2.6 | 3.3 |
| | 1050 | 404 | 6.8 | 3.4 |
| | 1475 | 400 | 10.4 | 3.4 |

As is apparent from Table 4 and Fig. 1, the disintegration time of tablets of Examples 1-1 through 1-3 is shortened as compared with that of Comparative Example 1. In addition, when the amount of erythritol incorporated into the tablet is increased, the disintegration time of a tablet becomes shorter.

### Example 2

Ethenzamide and corn starch were added into a fluidized-bed granulation-drying machine on the basis of the formulation of Example 2 shown in Table 5, and mixed for three minutes. The resultant mixture was granulated by use of a 5 w/v% aqueous solution (200 ml) of hydroxypropylcellulose under the following conditions: spray pressure 1.5 kg/cm², spray solution rate 15 ml/minute. After being dried, the resultant granules were sieved by use of a 16-mesh sieve. Erythritol and magnesium stearate were added to the thus-sieved granules on the basis of the formulation of Example 2 shown in Table 5, and mixed. Subsequently, the mixture was prepared into tablets by use of a punch having a flat impact face (diameter: 10 mm) in a single tableting machine at three different tableting pressures (from low to high pressure). The weight of a tablet was 400 mg. In Comparative Examples 2-1 and 2-2, on the basis of the formulations shown in Table 5, tablets were produced in the same manner as in Example 2. The thus-produced tablets were subjected to the disintegration test. The results are shown in Table 6 and Fig. 2.

**Table 5**

| Formulation | Example 2 | Comp. Ex. 2-1 | Comp. Ex. 2-2 |
|---|---|---|---|
| Ethenzamide | 250 | 250 | 250 |
| Corn starch | 56 | 136 | 56 |
| Erythritol | 80 | - | - |
| Low substituted hydroxypropylcellulose | - | - | 80 |
| Hydroxypropylcellulose | 10 | 10 | 10 |
| Magnesium stearate | 4 | 4 | 4 |
| Total | 400 | 400 | 400 |
| Note) In the formulation, unit is gram (g). | | | |

**Table 6**

| | Tableting pressure | Weight (g) | Hardness (kg) | Disintegration time (minute) |
|---|---|---|---|---|
| Example 2 | 525 | 404 | 4.1 | 1.7 |
| | 1040 | 405 | 8.1 | 2.5 |
| | 1550 | 406 | 11.8 | 3.3 |
| Comparative Example 2-1 | 515 | 401 | 3.5 | 14.6 |
| | 1035 | 400 | 8.2 | 18.7 |
| | 1550 | 403 | 12.3 | 13.7 |
| Comparative Example 2-2 | 540 | 409 | 3.0 | 2.4 |
| | 1065 | 404 | 7.9 | 4.2 |
| | 1530 | 404 | 11.2 | 7.0 |

As is apparent from Table 6 and Fig. 2, the disintegration time of tablets of Example 2 is shortened as compared with that of Comparative Example 2-1. In addition, the disintegration time of tablets of Example 2 is equal to or shorter than that required for tablets of Comparative Example 2-2 in which a water-swellable disintegrant, low substituted hydroxypropylcellulose, is incorporated.

### Example 3

Tranexamic acid and corn starch were added into a fluidized-bed granulation-drying machine on the basis of the formulation of Example 3 shown in Table 7, and were mixed for three minutes. The resultant mixture was granulated by use of a 5 w/v% aqueous solution (100 ml) of polyvinyl alcohol (partially hydrolyzed, PVA₂₀₅ₛ, product of Kuraray Co., Ltd.) under the following conditions: spray pressure 1.5 kg/cm², spray solution rate 15 ml/minute. After being dried, the resultant granules were sieved by use of a 16-mesh sieve. Erythritol and magnesium stearate were added to the thus-sieved granules on the basis of the formulation of Example 3 shown in Table 7, and mixed. Subsequently, the mixture was prepared into tablets by use of a punch having a flat impact face (diameter: 10 mm) in a single tableting machine at three different tableting pressures (from low to high pressure). The weight of a tablet was 400 mg. In Comparative Examples 3-1 and 3-2, on the basis of the formulations shown in Table 7, tablets were produced in the same manner as in Example 3. The thus-produced tablets were subjected to the disintegration test. The results are shown in Table 8 and Fig. 3.

**Table 7**

| Formulation | Example 3 | Comp. Ex. 3-1 | Comp. Ex. 3-2 |
|---|---|---|---|
| Tranexamic acid | 250 | 250 | 250 |
| Corn starch | 63 | 143 | 63 |
| Erythritol | 80 | - | - |
| Low substituted hydroxypropylcellulose | - | - | 80 |
| Hydroxypropylcellulose | 5 | 5 | 5 |
| Magnesium stearate | 2 | 2 | 2 |
| Total | 400 | 400 | 400 |
| Note) In the formulation, unit is gram (g). | | | |

**Table 8**

| | Tableting pressure (kg/punching) | Weight (g) | Hardness (kg) | Disintegration time (minute) |
|---|---|---|---|---|
| Example 3 | 530 | 403 | 1.3 | 1.3 |
| | 1015 | 407 | 2.6 | 0.9 |
| | 1530 | 413 | 3.7 | 1.5 |
| Comparative Example 3-1 | 500 | 401 | 1.3 | 2.6 |
| | 1015 | 403 | 3.0 | 3.1 |
| | 1515 | 408 | 4.5 | 3.7 |
| Comparative Example 3-2 | 510 | 401 | 2.6 | 0.9 |
| | 1030 | 402 | 5.7 | 1.9 |
| | 1535 | 406 | 8.0 | 3.4 |

As is apparent from Table 8 and Fig. 3, the disintegration time of tablets of Example 3 is shortened as compared with that of Comparative Example 3-1. In addition, the disintegration time of tablets of Example 3 is equal to that required for tablets of Comparative Example 3-2 in which a water-swellable disintegrant, low substituted hydroxypropylcellulose, is incorporated.

### Example 4

Erythritol and corn starch were added into a fluidized-bed granulation-drying machine on the basis of the formulation of Example 4 shown in Table 9, and were mixed for three minutes. The resultant mixture was granulated by use of water (800 ml) under the following conditions: spray pressure 2.0 kg/cm², spray solution rate 20 ml/minute. After being dried, the resultant granules were sieved by use of a 16-mesh sieve. Magnesium stearate (0.5 wt.%) was added to the thus-sieved granules and mixed. Subsequently, the mixture was prepared into tablets by use of a punch having a flat impact face (diameter: 10 mm) in a single tableting machine at a tableting pressure of 300-1300 kg/punching. The weight of a tablet was 400 mg. The thus-obtained tablets were subjected to the disintegration test and the disintegration test in the oral cavity. The results are shown in Table 10.

### Example 5

The procedure of Example 4 was repeated, except that erythritol was replaced by trehalose (hydrous crystal, product of Hayashibara Shoji Inc.) on the basis of the formulation of Example 5 shown in Table 9, to thereby obtain tablets. The thus-obtained tablets were subjected to the disintegration test and the disintegration test in the oral cavity. The results are shown in Table 10.

### Example 6

The procedure of Example 4 was repeated, except that erythritol was replaced by xylitol (product of Towa Chemical Industry Co., Ltd.) on the basis of the formulation of Example 6 shown in Table 9, to thereby obtain tablets. The thus-obtained tablets were subjected to the disintegration test and the disintegration test in the oral cavity. The results are shown in Table 10.

### Example 7

The procedure of Example 4 was repeated, except that erythritol was replaced by maltose (anhydrous crystal, product of Nihon Shokuhin Kako Co., Ltd.) on the basis of the formulation of Example 7 shown in Table 9, to thereby obtain tablets. The thus-obtained tablets were subjected to the disintegration test and the disintegration test in the oral cavity. The results are shown in Table 10.

### Comparative Example 4

The procedure of Example 4 was repeated, except that erythritol was replaced by D-sorbitol (product of Towa Chemical Industry Co., Ltd.) on the basis of the formulation of Comparative Example 4 shown in Table 9, to thereby obtain tablets. The thus-obtained tablets were subjected to the disintegration test and the disintegration test in the oral cavity. The results are shown in Table 10.

### Comparative Example 5

The procedure of Example 4 was repeated, except that erythritol was replaced by hydrogenated maltose starch syrup (product of Towa Chemical Industry Co., Ltd.) on the basis of the formulation of Comparative Example 5 shown in Table 9, to thereby obtain tablets. The thus-obtained tablets were subjected to the disintegration test and the disintegration test in the oral cavity. The results are shown in Table 10.

**Table 9**

| Formulation | Ex. 4 | Ex. 5 | Ex. 6 | Ex. 7 | Comp. Ex. 4 | Comp. Ex. 5 |
|---|---|---|---|---|---|---|
| Corn starch | 240 | 240 | 240 | 240 | 240 | 240 |
| Erythritol | 560 | - | - | - | - | - |
| Trehalose | - | 560 | - | - | - | - |
| Xylitol | - | - | 560 | - | - | - |
| Maltose | - | - | - | 560 | - | - |
| D-Sorbitol | - | - | - | - | 560 | - |
| Hydrogenated maltose starch syrup | - | - | - | - | - | 560 |
| Total | 800 | 800 | 800 | 800 | 800 | 800 |
| Note) In the formulation, unit is gram (g). | | | | | | |

**Table 10**

| | Tableting pressure (kg/punching) | Weight (g) | Hardness (kg) | Disintegration time (second) | Disintegration time in the oral cavity (second) |
|---|---|---|---|---|---|
| Ex. 4 | 1091 | 400 | 4.0 | 16 | 15-22 |
| Ex. 5 | 450 | 412 | 2.8 | 53 | 50-60 |
| Ex. 6 | 850 | 400 | 1.8 | 43 | 51-78 |
| Ex. 7 | 730 | 398 | 0.6 | 40 | 21-26 |
| Comp. Ex. 4 | 1100 | 406 | 2.0 | 46 | 70-145 |
| Comp. Ex. 4 | 960 | 412 | 2.3 | 108 | 72-159 |

As is apparent from Table 10, the disintegration time of tablets of Example 4 through 7 is relatively shorter than that required for tablets of Comparative Examples 4 and 5. Particularly, the disintegration time of tablets of Example 4 through 7 in the oral cavity is considerably shortened. Thus, erythritol, trehalose, xylitol, and maltose may be an excellent disintegrant to be employed in a solid composition which is rapidly disintegrated in the oral cavity.

### Example 8

Ethenzamide, corn starch, and erythritol were added into a fluidized-bed granulation-drying machine on the basis of the formulation of Example 8 shown in Table 11, and were mixed for three minutes. The resultant mixture was granulated by use of a 5 w/v% aqueous solution (200 ml) of hydroxypropylcellulose under the following conditions: spray pressure 1.5 kg/cm², spray solution rate 15 ml/minute. After being dried, the resultant granules were sieved by use of a 16-mesh sieve. Magnesium stearate were added to the thus-sieved granules on the basis of the formulation of Example 8 shown in Table 11, and mixed. Subsequently, the mixture was prepared into tablets by use of a punch having a flat impact face (diameter: 10 mm) in a single tableting machine at a tableting pressure of 100-800 kg/punching. The weight of a tablet was 400 mg. The thus-obtained tablets were subjected to the disintegration test and stability tests under moisture absorption conditions. The results are shown in Tables 12 and 13.

### Example 9

The procedure of Example 8 was repeated, except that erythritol was replaced by trehalose on the basis of the formulation of Example 9 shown in Table 11, to thereby obtain tablets. The thus-obtained tablets were subjected to the disintegration test and stability tests under moisture absorption conditions. The results are shown in Tables 12 and 13.

### Comparative Example 6

The procedure of Example 8 was repeated, except that erythritol was replaced by croscarmellose sodium on the basis of the formulation of Comparative Example 6 shown in Table 11, to thereby obtain tablets. The thus-obtained tablets were subjected to the disintegration test and stability tests under moisture absorption conditions. The results are shown in Tables 12 and 13.

**Table 11**

| Formulation | Example 8 | Example 9 | Comparative Example 6 |
|---|---|---|---|
| Ethenzamide | 250 | 250 | 250 |
| Corn starch | 56 | 56 | 116 |
| Erythritol | 80 | - | - |
| Trehalose | - | 80 | - |
| Croscarmellose sodium | - | - | 20 |
| Hydroxypropylcellulose | 10 | 10 | 10 |
| Magnesium stearate | 4 | 4 | 4 |
| Total | 400 | 400 | 400 |
| Note) In the formulation, unit is gram (g). | | | |

**Table 12**

| | | Tableting pressure (kg/punching) | Weight (g) | Hardness (kg) | Disintegrating time (second) |
|---|---|---|---|---|---|
| Example 8 | (1) | 390 | 405 | 7.7 | 2.0 |
| | (2) | 680 | 401 | 11.8 | 2.5 |
| Example 9 | (1) | 150 | 409 | 3.9 | 7.8 |
| | (2) | 300 | 408 | 7.0 | 11.7 |
| Comp. Ex. 6 | (1) | 290 | 407 | 3.5 | 11.9 |
| | (2) | 600 | 409 | 7.3 | 11.1 |

**Table 13**

| | | Initial | 25°C, 75% open air, 7 days | 40°C, 75% open air, 7 days |
|---|---|---|---|---|
| Example 8-(2) | Hardness (kg) | 11.8 | 11.0 | 9.6 |
| | Disintegration time (minute) | 2.5 | 2.5 | 2.5 |
| | Increase in weight by moisture absorption (%) | - | 1.3 | 0.4 |
| | Tablet size (mm) | 10.06 | 10.12 | 10.12 |
| | Tablet thickness (mm) | 4.56 | 4.65 | 4.70 |
| | Percentage of increase in volume (%) | - | 3.3 | 3.7 |
| Example 9-(2) | Hardness (kg) | 7.0 | 6.2 | 4.8 |
| | Disintegration time (minute) | 11.7 | 8.3 | 10.2 |
| | Increase in weight by moisture absorption (%) | - | 1.2 | 0.4 |
| | Tablet size (mm) | 10.06 | 10.02 | 10.10 |
| | Tablet thickness (mm) | 4.97 | 5.03 | 5.04 |
| | Percentage of increase in volume (%) | - | 0.4 | 2.1 |
| Comparative Example 6-(2) | Hardness (kg) | 7.3 | 4.3 | 3.2 |
| | Disintegration time (minute) | 11.1 | 10.7 | 10.9 |
| | Increase in weight by moisture absorption (%) | - | 3.0 | 1.5 |
| | Tablet size (mm) | 10.09 | 10.28 | 10.25 |
| | Tablet thickness (mm) | 4.82 | 5.06 | 5.04 |
| | Percentage of increase in volume (%) | - | 9.0 | 7.9 |

As is apparent from Table 12, the disintegration time of tablets of Examples 8 and 9 is equal to or shorter than that required for tablets of Comparative Example 6 in which a water-swellable disintegrant, croscarmellose sodium, is incorporated. As is apparent from Table 13, tablets of Examples 8 and 9 exhibit excellent stability as compared with those of Comparative Example 6. Namely, in the tablets of Examples 8 and 9, reduction in hardness, increase in weight by moisture absorption, and percentage of increase in volume are small as compared with those of Comparative Example 6. In the tablets of Examples 8 and 9, slight increase in volume is attributed not to erythritol and trehalose which are not hygroscopic, but to corn starch incorporated into the tablets, which absorbs moisture.

### Industrial Applicability

The disintegration time of a solid composition comprising the disintegrant of the present invention is equal to or shorter than that comprising a conventionally-used water-swellable disintegrant. The disintegrant of the present invention exhibits no swellability, and thus increase in volume of the agent is not observed with passage of time. Particularly, when the agent is incorporated into a film-coated or sugar-coated tablet, stability of the tablet over time is enhanced. When the disintegrant of the present invention is incorporated into powders, granules, chewable tablets, or shaped products which rapidly disintegrate or dissolve in the oral cavity, such a solid composition containing the agent can provide a favorable sensation on oral administration, since the composition does not provide a gritty sensation in the oral cavity and the composition dissolves rapidly in the oral cavity. A solid composition comprising the disintegrant of the present invention does not require a complicated production process comprising a number of steps. Namely, the composition can be produced through a general production process, resulting in low cost and high productivity.

## Claims

1. A disintegrant comprising a substance which is solid at room temperature and has a water solubility of 30 wt.% or more at 37°C, a saturated aqueous solution of the substance having a viscosity of 50 mPa·s or less at 37°C.

2. A disintegrant containing one or more substances selected from the group consisting of erythritol, trehalose, xylitol, and maltose.

3. A solid composition containing a disintegrant as described in claim 1 or 2.

4. A solid composition containing a disintegrant comprising a substance which is solid at room temperature and has a water solubility of 30 wt.% or more at 37°C, a saturated aqueous solution of the substance having a viscosity of 50 mPa·s or less at 37°C, wherein the amount of the disintegrant is 5-99 wt.% with respect to the total weight of the solid composition.

5. A solid composition containing a disintegrant containing one or more substances selected from the group consisting of erythritol, trehalose, xylitol, and maltose, wherein the amount of the disintegrant is 5-99 wt.% with respect to the total weight of the solid composition.
